# EUROPEAN PATENT APPLICATION

(11) **EP 4 289 392 A1**
(43) Date of publication of application: **13.12.2023**
(21) Application number: 22750097.2
(22) Date of filing: 08.02.2022
(51) Int. Cl.: A61C 9/00, A61B 5/00, G06T 19/20, G16H 30/00, A61C 8/00, A61B 18/20

(54) **DATA PROCESSING METHOD**

(30) Priority: 08.02.2021 KR 20210017681
(71) Applicant: Medit Corp., Seoul 07207 (KR)
(72) Inventor: SONG, Myoung Woo, Seoul 02842 (KR); KIM, Jin Young, Seoul 02842 (KR)
(74) Representative: Maiwald GmbH
(86) International application number: PCT/KR2022/001924
(87) International publication number: WO 2022/169348

(57) **Abstract**

A data processing method according to the present invention comprises the steps of: acquiring pieces of scan data from different models; aligning the acquired pieces of scan data; and selectively combining a portion of one of the aligned pieces of scan data with another of the aligned pieces of scan data.

## Description

### TECHNICAL FIELD

The present disclosure relates to a data processing method and, more particularly, to a data processing method for selectively merging scan data, which facilitates identification of margin lines of a preparation tooth, or the like, with scan data that resembles an actual oral cavity.

### BACKGROUND

In order to design and fabricate a dental prosthesis (such as an implant) in a patient's oral cavity, information about the margin line of the patient's preparation tooth is required. In order to obtain the preparation tooth margin line information, a user (e.g., a dentist) may scan the inside of the patient's actual oral cavity, or may scan a plaster cast obtained by taking an impression of the patient's oral cavity with alginate or the like. The user may scan the plaster cast instead of the patient's actual oral cavity in order to perform free and precise scanning from multiple angles.

However, even when scanning a plaster cast, if a margin line of the preparation tooth is formed in a subgingival region, it is difficult for the user to obtain information about the margin line due to narrow gaps and deep troughs between teeth of the plaster cast. In the scanning process, when a table scanner, which has a wide field of view but cannot perform detailed scanning, is used, it is very difficult to scan a portion to be trimmed.

In order to obtain margin line information, a plaster cast may be trimmed. In this case, a portion of the plaster model that is removed in the trimming process is also necessary for designing a dental prosthesis (such as an implant). However, as described above, scanning the plaster model before the trimming process as a whole has the disadvantage of reducing the precision, and segmenting and partially scanning the plaster model before the trimming process has the disadvantage of increasing the work time and decreasing the work efficiency.

### SUMMARY

In order to solve the above-described problems, the present disclosure provides a data processing method capable of complementing a portion of an oral model before trimming from an impression-taking cast.

The technical tasks of the present disclosure are not limited to the above-described technical tasks, and other technical tasks, which have not been described above, will be clearly understood by a person skilled in the art from the following description.

In order to solve the above problems, a data processing method according to the present disclosure includes operations of obtaining scan data from different casts; aligning the obtained scan data; and selectively merging a portion of one piece of the aligned scan data with another piece of the aligned scan data.

Further, the scan data may include pre-scan data obtained by scanning a first cast, and second scan data obtained by scanning a second cast generated based on the first cast.

Further, the first cast may be an impression-taking cast, and the second cast may be an oral cavity cast.

Further, a part of a gingival portion of the second cast may be formed by trimming.

Further, in the aligning operation, first scan data obtained by inverting the pre-scan data may be aligned with the second scan data.

Further, the operation of selectively merging of the scan data may include operations of measuring an intersection distance between a predetermined region of the first scan data obtained by inverting the pre-scan data and a predetermined region of the second scan data; selecting at least a portion of the first scan data based on the intersection distance; and combining the data selected in the selecting operation, wherein the selected data corresponds to a trimmed portion of the second scan data indicating a trimmed portion of the second cast.

Further, the intersection distance may be a distance when a ray generated in a normal direction from one of the first scan data and the second scan data meets the other.

Further, in the selecting operation, in case that the intersection distance is less than a first threshold value, the first scan data of a corresponding portion may be deleted, and in case that the intersection distance is equal to or greater than the first threshold value, the first scan data of a corresponding portion may be selected.

Further, in the selecting operation, in case that the intersection distance is less than the first threshold value or equal to or greater than a second threshold value, the first scan data of a corresponding portion may be deleted, and in case that the intersection distance is equal to or greater than the first threshold value and less than the second threshold value, the first scan data of a corresponding portion may be selected.

Further, in the combining operation, the first scan data selected in the selecting operation and the second scan data may be combined with each other to generate final scan data, and the final scan data may be formed to display at least one of the selected first scan data and the second scan data.

Further, the operation of selectively merging the scan data may include operations of designating an intersection reference region of the second scan data; generating at least one ray in a normal direction from first scan data obtained by inverting the pre-scan data; selecting portions of the first scan data where the ray meets the intersection reference region; and combining the second scan data with the portions of the first scan data selected in the selecting operation, wherein the portions of the first scan data correspond to trimmed portions of the second scan data indicating trimmed portions of the second cast.

Further, in the combining operation, the portions of the first scan data selected in the selecting operation and the second scan data may be combined with each other to generate final scan data, and the final scan data may be formed to display at least one of the selected first scan data and the second scan data.

Further, the portions of the first scan data may be portions between a first portion of the first scan data, at which the ray meets one end of the intersection reference region, and a second portion of the first scan data, at which the ray meets the other end of the intersection reference region.

According to the present disclosure, there is an advantage of obtaining precise margin line information from a trimmed oral cavity cast and obtaining a pre-trimming shape from an impression-taking cast, so that a user can selectively identify required data.

Further, according to the present disclosure, there is an advantage in that first scan data and second scan data are combined with each other to obtain final scan data that accurately represents the actual oral cavity of a patient.

### BRIEF DESCRIPTION OF DRAWINGS

FIG. 1 illustrates a margin line to be obtained in a data processing method according to the present disclosure.
FIG. 2 is a flowchart of a data processing method according to the present disclosure.
FIG. 3 is a detailed flowchart of operation S130 in FIG. 2.
FIGS. 4A and 4B illustrate pre-scan data and first scan data.
FIG. 5 illustrates an oral cavity cast.
FIG. 6 illustrates a segment of an oral cavity cast.
FIG. 7 illustrates a process for trimming a segment of an oral cavity cast.
FIG. 8 is second scan data obtained by scanning an assembled oral cavity cast after trimming.
FIG. 9 illustrates a process of aligning the first scan data with the second scan data.
FIG. 10 is an enlarged view of portion A in FIG. 9.
FIG. 11 is an enlarged view of portion B in FIG. 9.
FIG. 12 illustrates final scan data.
FIG. 13 illustrates a second scan data model.
FIG. 14 illustrates a pre-scan data model.
FIG. 15 illustrates some of portions that are deleted when selectively merging the first scan data and the second scan data.
FIG. 16 illustrates a final scan data model.
FIG. 17 is a detailed flowchart of operation S230 of a data processing method according to another embodiment of the present disclosure.
FIG. 18 illustrates a process for designating an intersection reference region in the second scan data.
FIG. 19 illustrates a process of aligning the first scan data with the second scan data.
FIG. 20 illustrates a process of selecting a portion of the first scan data in the intersection reference region.
FIG. 21 schematically illustrates a configuration of a data processing device performing a data processing method according to the present disclosure.

### [Description of symbols]

| | | | |
|---|---|---|---|
| 10: | Oral cavity cast | 20: | Pre-scan data |
| 30: | First scan data | 40: | Second scan data |
| 50: | Final scan data | | |

### DETAILED DESCRIPTION

Hereinafter, some embodiments of the present disclosure will be described in detail with reference to the accompanying drawings. It should be noted that in adding reference numerals to elements in each drawing, the same elements will be designated by the same reference numerals, if possible, although they are illustrated in different drawings. Furthermore, in the following description of embodiments of the present disclosure, a detailed description of known functions and configurations incorporated herein will be omitted when it is determined that the description interferes with the understanding of the embodiments of the present disclosure.

Terms, such as first, second, A, B, (a), (b) or the like may be used herein when describing elements of embodiments of the present disclosure. These terms are merely used to distinguish one element from other elements, and a property, an order, a sequence, and the like of a corresponding element are not limited by the terms. Furthermore, unless otherwise defined, all terms used herein, including technical or scientific terms, have the same meanings as those generally understood by a person skilled in the art to which the present disclosure pertains. Such terms as those defined in a generally used dictionary should be interpreted to have meanings equal to the contextual meanings in the relevant field of art, and are not to be interpreted to have ideal or excessively formal meanings unless clearly defined in the present disclosure.

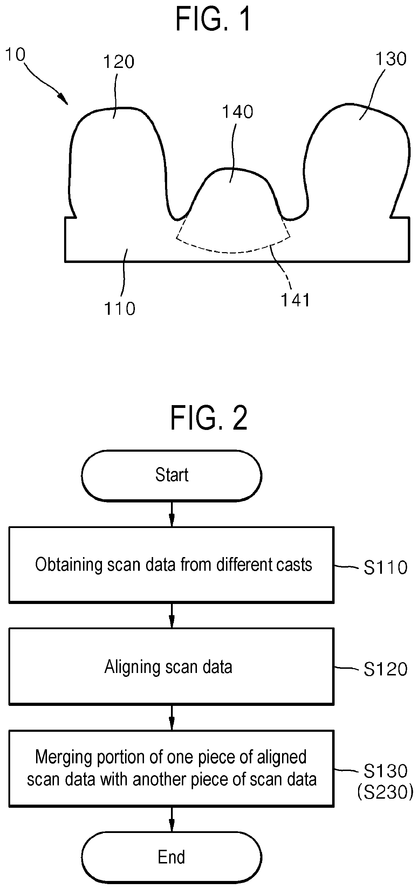

FIG. 1 illustrates a margin line 141 to be obtained in a data processing method according to the present disclosure.

FIG. 1 illustrates an oral cavity cast 10 (also referred to as a second cast in the specification) of a patient's oral cavity, obtained through an impression-taking process. For example, the oral cavity cast has a gingival portion 110, tooth portions 120 and 130, and a preparation tooth portion 140. The preparation tooth portion 140 represents a preparation tooth, and the preparation tooth portion 140 may have a margin line 141 inside the gingival portion 110. The present disclosure provides a data processing method in which information about the margin line 141 can be easily obtained by trimming the oral cavity cast 10, and the pre-trimming shape of the gingival portion 140 can be obtained from an impression-taking cast which will be described later.

Hereinafter, a data processing method according to one embodiment of the present disclosure will be described in detail.

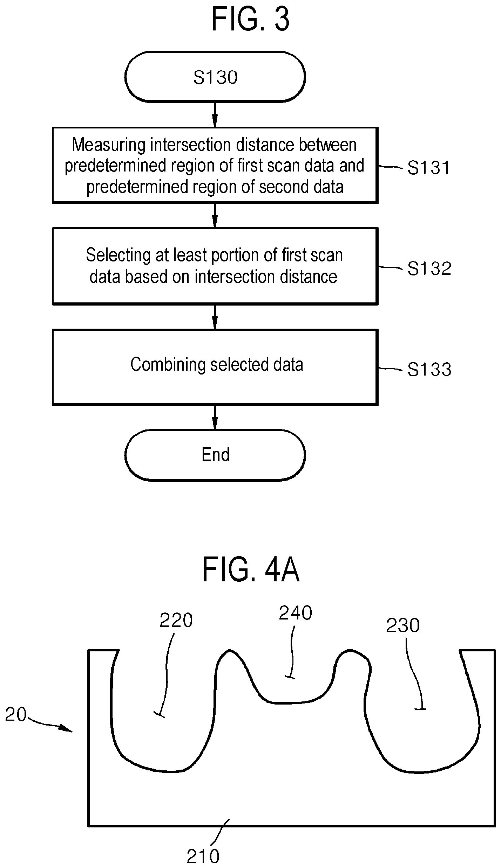

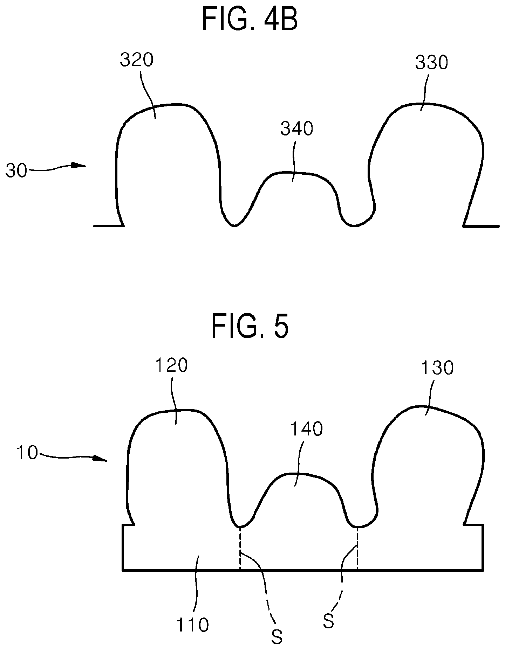

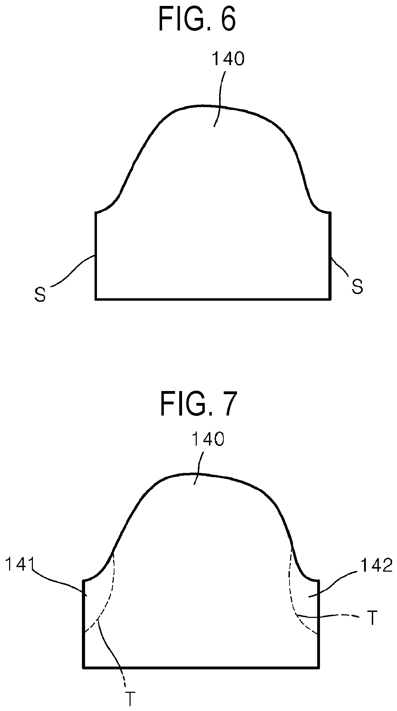

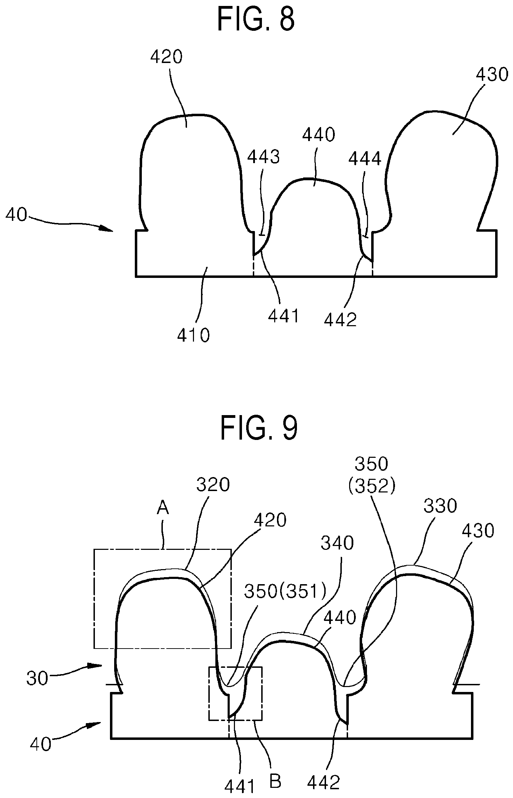

FIG. 2 is a flowchart of a data processing method according to the present disclosure. FIG. 3 is a detailed flowchart of operation S130 in FIG. 2. FIGS. 4A and 4B illustrate pre-scan data 20 and first scan data 30. FIG. 5 illustrates the oral cavity cast 10. FIG. 6 illustrates a segment of the oral cavity cast 10. FIG. 7 illustrates a process for trimming a segment of the oral cavity cast 10. FIG. 8 illustrates second scan data obtained by scanning an assembled oral cavity cast 10 after trimming.

Referring to FIGS. 2 to 8 overall, the data processing method according to the present disclosure includes an operation S110 of obtaining scan data 20, 30, and 40 from different casts via a scan unit. In the operation S110 of obtaining the scan data, the scan data 20, 30, and 40 may be obtained from at least two different casts, respectively. At this time, the scan data 20, 30, and 40 may include pre-scan data 20 obtained by scanning a first cast, and second scan data 40 obtained by scanning a second cast. Further, the second cast may be generated based on the first cast. For example, the second cast may be an oral cavity cast obtained by pouring plaster into an impression-taking cast and solidifying the plaster.

Referring to FIG. 4A, the first cast may be an impression-taking cast in which the actual interior of a patient's oral cavity is recorded in a negative shape. The first cast may be obtained in a shape in which an impression-taking material is recessed to correspond to the protruding shape of the teeth and the protruding shape of the gingiva. A user may scan the first cast to obtain the pre-scan data 20.

The pre-scan data 20 may include a gingival portion 210, normal tooth portions 220 and 230, and a preparation tooth portion 240. A preparation tooth may be a portion of a tooth that has been ground through a grinding process in order to apply a treatment prosthesis, or the like. Accordingly, the preparation tooth may have a lower height than a normal tooth, and the preparation tooth portion 240 may be less recessed than the normal tooth portions 220 and 230.

Meanwhile, referring to FIG. 4B, the first cast is formed in a negative shape, and the pre-scan data 20 also has a negative shape because the pre-scan data 20 has been obtained by scanning the first cast. However, in order to align the pre-scan data 20 with the second scan data 40 which will be described later, the pre-scan data 20 must be converted into a shape corresponding to the second scan data 40. Thus, the pre-scan data 20 having a negative shape may be inverted to have a positive shape. At this time, a vertex normal method may be used as a method for inverting the pre-scan data 20, and the first scan data 30 may be obtained from the pre-scan data 20 by the above method.

Further, for example, the scan unit may be a three-dimensional scanner capable of photographing a cast to obtain the scan data 20, 30, and 40 of the cast. More specifically, the three-dimensional scanner may be a handheld scanner that is held by a user to freely photograph a cast from various distances and angles with respect to the cast. However, the scan unit is not limited thereto, and may also be a table scanner wherein a cast is placed on a tray and the cast is rotated or tilted to obtain the scan data 20, 30, and 40.

Hereinafter, a process of obtaining the second scan data 40 will be described.

Referring to FIG. 5, in order to trim the oral cavity cast 10 between the preparation tooth portion 140 and the normal tooth portions 120 and 130, the user may segment the oral cavity cast 10 between the preparation tooth portion 140 and the normal tooth portions 120 and 130. At this time, the user may generate multiple segments of the oral cavity cast 10 by sawing segment regions S between the preparation tooth portion 140 and the normal tooth portions 120 and 130. The segment regions S may be spaced apart from each other by a predetermined distance relative to the preparation tooth portion 140. The distance at which the segmented regions S are formed is variable, and the user may saw regions as deemed appropriate to facilitate obtaining margin line information.

Referring to FIG. 6, a segment portion of the oral cavity cast 10 produced by sawing is illustrated. A part of the preparation tooth portion 140 formed between the segment regions S may be deleted by a trimming process which will be described later. Alternatively, a user may scan and combine the multiple segments of the oral cavity cast 10. However, the process of scanning and combining the segments of the oral cavity cast 10 requires a large amount of time and system computation, and it is preferable to trim some of the segments of the oral cavity cast 10 and then combine the segments of the oral cavity cast 10 again to scan the entire oral cavity cast 10.

Hereinafter, the trimming process will be described with reference to FIG. 7. In a segment of the oral cavity cast 10, the gingival portion 110 may be partially ground to be adjacent to the segment regions S of the preparation tooth portion 140. This grinding process is referred to as a trimming process. Through the trimming process, both ends 141 and 142 of the preparation tooth portion 140 may be removed to allow the user to more easily identify the margin line. As illustrated in FIG. 7, portions to be trimmed are depicted as being both ends 141 and 142 of the preparation tooth portion 140, but are not necessarily limited thereto. In practice, it should be interpreted that the portions to be trimmed are the outer circumference surface of the preparation tooth portion 140.

When the oral cavity cast 10 is segmented and then the trimming process is performed, the user may recombine the segments of the oral cavity cast 10. Further, the user may obtain the second scan data 40 by scanning the combined oral cavity cast 10 by means of the scan unit. That is, the second scan data 40 may be digital data of the second cast. The second scan data 40 may include a gingival portion 410, normal tooth portions 420 and 430, and a preparation tooth portion 440. Furthermore, at both ends of the preparation tooth portion 440, trimmed portions 441 and 442 are formed corresponding to both ends trimmed after segmenting the oral cavity cast 10 described above, and the gingival portion 410 is deleted to reveal trimming spaces 443 and 444. Even when the margin line information is easily obtained through the trimmed portions 441 and 442, the gingival portion 410 without the trimming spaces 443 and 444 before trimming is also necessary for fabrication of a treatment prosthesis. However, in the entire oral cavity cast 10 before trimming, it is difficult to scan spaces between the normal tooth portions 120 and 130 and the preparation tooth portion 140, and in the segment of the oral cavity cast 10 before trimming, the amount of system computation for data combination after scanning may be excessive. Thus, to solve the above problems, the pre-scan data 20 and/or the first scan data 30 obtained by scanning the impression-taking cast may be used.

The following describes a process for aligning the first scan data 30 with the second scan data 40.

FIG. 9 illustrates the process of aligning the first scan data 30 with the second scan data 40.

Referring to FIGS. 2 and 9, in order to complement the trimming spaces 443 and 444 of the second scan data 40, the data processing method according to the present disclosure may include an operation S120 of aligning the obtained scan data 20, 30, and 40. For example, in the aligning operation S120, the first scan data 30 and the second scan data 40 may be aligned in order to align the scan data 20, 30, and 40 having corresponding shapes. In order to align the first scan data 30 with the second scan data 40, the two pieces of data may be aligned with each other in a way that minimizes the deviation between a predetermined region of the first scan data 30 and a predetermined region of the second scan data 40. To align the first scan data 30 with the second scan data 40, an iterative closest point (ICP) method may be used. However, the present disclosure is not limited thereto, and at least one of various data alignment methods for aligning the first scan data 30 with the second scan data 40 so as to minimize the deviation therebetween may be used. By aligning the first scan data 30 with the second scan data 40, normal tooth portions 320 and 330 of the first scan data 30 are aligned with the normal tooth portions 420 and 430 of the second scan data 40, and a preparation tooth portion 340 of the first scan data 30 is aligned with the preparation tooth portion 440 of the second scan data 40. Further, the trimmed portions 441 and 442 of the second scan data 40 are aligned with gingival complementary portions 350 of the first scan data 30. A first gingival complementary portion 351 of the gingival complementary portions 350 may be aligned with the first trimmed portion 441, and a second gingival complementary portion 352 may be aligned with the second trimmed portion 442.

Hereinafter, a description will be made of an operation S130 of selectively merging the scan data 20, 30, and 40 in the data processing method according to the present disclosure.

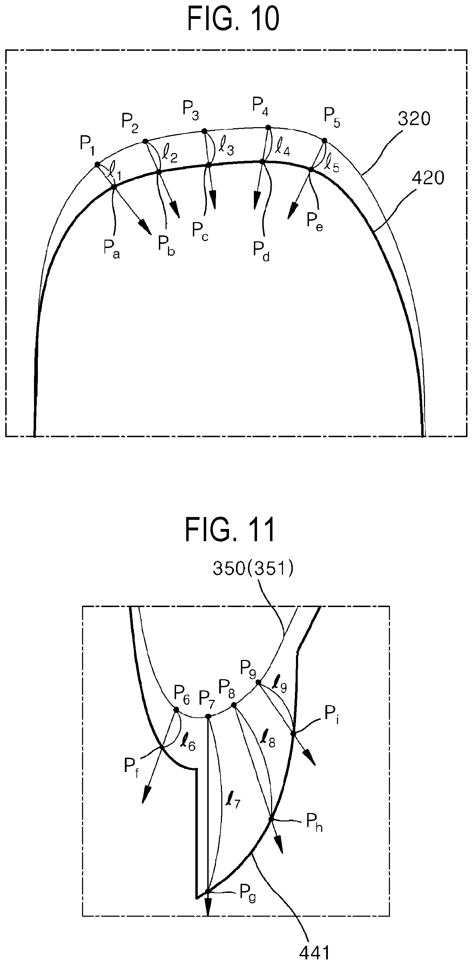

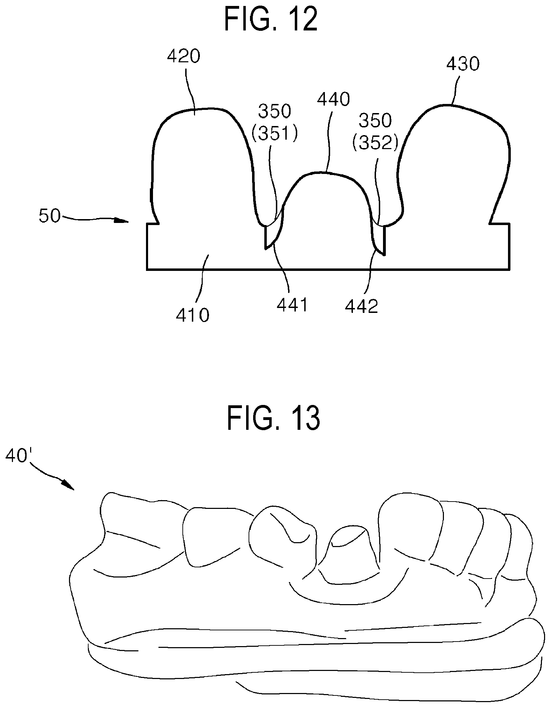

FIG. 9 illustrates a process of aligning the first scan data with the second scan data. FIG. 10 is an enlarged view of portion A in FIG. 8. FIG. 11 is an enlarged view of portion B in FIG. 8. FIG. 12 illustrates final scan data 50.

Referring to FIGS. 2, 3, and 9 to 11 overall, the data processing method according to the present disclosure includes the operation S130 of selectively merging a portion of one of the aligned scan data 30 and 40 with the other scan data. For example, the selectively merging operation S130 may be an operation of selectively merging a portion of the first scan data 30 with the second scan data 40.

It is possible to merge all of the first scan data 30 and the second scan data 40, but this may result in unnecessary waste of storage space and increased system computation, thereby causing user inconvenience. Further, a user may be only interested in the gingival complementary portions 350 before trimming of the trimmed portions 441 and 442, and in the case of portions other than the gingival complementary portions 350, the second scan data 40 may better correspond to the actual oral cavity of a patient. Thus, unnecessary portions of the aligned scan data 30 and 40 may not be deleted or selected, and only the necessary portions may be selectively merged to generate final scan data which will be described later.

The merging operation S130 will be described in more detail. The merging operation S130 includes an operation S131 of measuring an intersection distance between a predetermined region of the first scan data 30 obtained by inverting the pre-scan data and a predetermined region of the second scan data 40 obtained by scanning the second cast. More specifically, the first scan data 30 may refer to data in which the pre-scan data 20, having a negative shape and obtained by scanning the first cast, is converted into a positive shape by using the vertex normal method, as described above.

In order to measure the intersection distance between the predetermined region of the first scan data 30 and the predetermined region of the second scan data 40, a ray may be generated in a normal direction from at least one point included in the predetermined region of the first scan data 30 or the predetermined region of the second scan data 40. The predetermined region of the first scan data 30 may be at least one among a gingival portion 310, the normal tooth portions 320 and 330, the preparation tooth portion 340, and the gingival complementary portions 350. Further, the predetermined region of the second scan data 40 may be at least one among the gingival portion 410, the normal tooth portions 420 and 430, the preparation tooth portion 440, and the trimmed portions 441 and 442.

More specifically, a ray may be generated in a normal direction from at least one arbitrary point included in the predetermined region of the first scan data 30. As illustrated in FIG. 10, for example, a ray may be generated in a normal direction from each of five arbitrary points P1, P2, P3, P4, and P5 included in the first normal tooth portion 320 of the first scan data 30. In this case, the normal directions at the points P1, P2, P3, P4, and P5 of the first normal tooth portion 320 may be the normal directions of imaginary planes including the respective points P1, P2, P3, P4, and P5 and tangent to the first normal tooth portion 320. Each of the normal directions may be at least one of a buccal direction and a lingual direction opposite the buccal direction. More specifically, the normal directions may be directions extending bidirectionally or unidirectionally from the first scan data 30.

At least some of the rays generated from the points P1, P2, P3, P4, and P5 of the first normal tooth portion 320 in the first scan data 30 may meet the second scan data 40. For example, at least some of the rays generated from the points P1, P2, P3, P4, and P5 of the first normal tooth portion 320 in the first scan data 30 may meet points Pa, Pb, Pc, Pd, and Pe of the first normal tooth portion 420 in the second scan data 40. In this case, a first distance l1 between the point P1 and the point Pa, a second distance l2 between the point P2 and the point Pb, a third distance l3 between the point P3 and the point Pc, a fourth distance l4 between the point P4 and the point Pd, and a fifth distance l5 between the point P5 and the point Pe may be intersection distances. The intersection distances l1, l2, l3, l4, and l5 may be measured as straight-line distances. The intersection distances l1, l2, l3, l4, and l5 may be used as criteria for selecting a portion of the first scan data 30.

As illustrated in FIG. 11, in the gingival complementary portions 350 of the first scan data 30, rays are also generated from four points P6, P7, P8, and P9, and the generated rays may meet the trimmed portion 441 of the second scan data 40. In this case, a sixth distance l6 between the point P6 and a point Pf, a seventh distance l7 between the point P7 and a point Pg, an eighth distance l8 between the point P8 and a point Ph, and a ninth distance l9 between the point P9 and a point Pi may also be intersection distances.

The merging operation S130 may include an operation S132 for selecting at least a portion of the first scan data 30 based on the intersection distances l1, l2, l3, l4, 15,16, l7, l8, and l9. For example, the selecting operation S132 may be an operation for selecting the gingival complementary portions 350 in the first scan data 30. Thus, in order to select the gingival complementary portions 350, a first threshold value and a second threshold value are applied as determination criteria for the intersection distances L1, L2, L3, L4, L5, L6, L7, L8, and L9. At this time, the first threshold value may be less than the second threshold value.

As an example, in the selecting operation S132, only the first threshold value may be applied as the determination criterion for the intersection distances l1, l2, l3, l4, l5, l6, l7, l8, and l9. More specifically, in the selection operation S132, when the intersection distances l1, l2, l3, l4, l5, l6, l7, l8, and l9 are less than the first threshold value, corresponding portions of the first scan data 30 may be deleted. For example, as illustrated in FIG. 10, if the first distance l1, the second distance l2, the third distance l3, the fourth distance l4, and the fifth distance l5 are less than the first threshold value, corresponding portions of the first scan data 30 are deleted. In the second scan data 40, the normal tooth portions 420 and 430 do not need to be complemented by the normal tooth portions 320 and 330 of the first scan data 30.

Further, as illustrated in FIG. 11, the sixth distance l6 may be less than the first threshold value, and the seventh to ninth distances l7, l8, and l9 may be equal to or greater than the first threshold value. At this time, corresponding portions of the first scan data 30 may be selected. That is, the gingival complementary portions 350 may be selected and combined with the second scan data 40, and the gingival complementary portions 350 may correspond to the trimmed portions 441 and 442 of the second scan data 40, and may represent the pre-trimming shape of the trimmed portions 441 and 442.

As another example, in the selecting operation S132, the first threshold value and the second threshold value may be applied together as determination criteria for the intersection distances l1, l2, l3, l4, l5, l6, l7, l8, and l9. More specifically, in the selecting operation S132, when the intersection distances l1, l2, l3, l4, l5, l6, l7, l8, and l9 are less than the first threshold value, corresponding portions of the first scan data 30 may be deleted. For example, as illustrated in FIG. 10, when the first distance l1, the second distance l2, the third distance l3, the fourth distance l4, and the fifth distance l5 are less than the first threshold value, corresponding portions of the first scan data 30 are deleted. In the second scan data 40, the normal tooth portions 420 and 430 do not need to be complemented by the normal tooth portions 320 and 330 of the first scan data 30.

Further, as illustrated in FIG. 11, the sixth distance L6 may be less than the first threshold value, and the seventh to ninth distances l7, l8, and l9 may be equal to or greater than the first threshold value and less than the second threshold value. At this time, corresponding portions of the first scan data 30 may be selected. That is, the gingival complementary portions 350 may be selected and combined with the second scan data 40, and the gingival complementary portions 350 may correspond to the trimmed portions 441 and 442 of the second scan data 40, and may represent the pre-trimming shape of the trimmed portions 441 and 442.

When the intersection distances l1, l2, l3, l4, l5, l6, l7, l8, and l9 are greater than or equal to the second threshold value, no intersection occurs between the first scan data 30 and the second scan data 40 (intersection distance infinity), or the region from which a ray is generated may be soft tissue or a noise data portion. Therefore, when the intersection distances l1, l2, l3, l4, 15, l6, l7, l8, and l9 are greater than or equal to the second threshold value, the first scan data 30 may be deleted. In this way, by selecting only a portion of the first scan data 30 at which an intersection distance is within a predetermined range and combining the selected portion with the second scan data 40, unnecessary waste of data storage space may be avoided and the system computation amount may be reduced.

According to the foregoing, it has been described that rays are generated from nine arbitrary points in the first scan data 30 and the intersection distances are measured, but the present disclosure is not necessarily limited thereto. That is, the number of points may be increased or decreased to any suitable number depending on the computation ability of the system and the needs of the user.

Referring to FIGS. 3 and 12, the merging operation S130 may further include an operation S133 of combining the data selected in the selecting operation S132. That is, in the combining operation S133, final scan data 50 may be generated by combining at least a portion of the first scan data 30 selected in the above-described selecting operation S132 with at least a portion of the second scan data 40. For example, the final scan data 50 may be generated by combining all of the second scan data 40 with the gingival complementary portions 350 of the first scan data 30. The gingival complementary portions 350 of the first scan data 30 may be portions of the first scan data 30 that have been selected without being deleted in the above-described selecting operation S132. By obtaining the final scan data 50, the user may identify only the second scan data 40, in which the trimmed portions 441 and 442 are displayed, to obtain margin line information as needed, or may identify the combined form of the first scan data 30, in which the gingival complementary portions 350 including the first gingival complementary portion 351 and the second gingival complementary portion 352 are displayed, and the second scan data 40. Thus, there is an advantage that the user can provide an optimal treatment prosthesis for a patient through the final scan data 50.

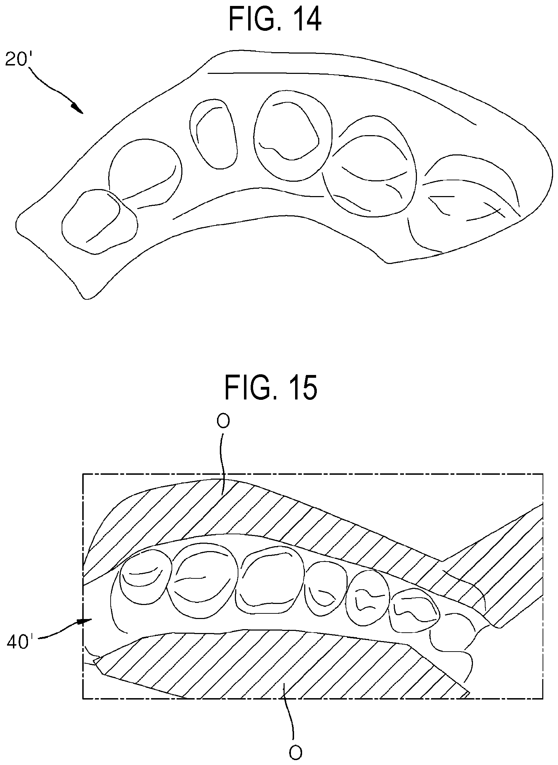

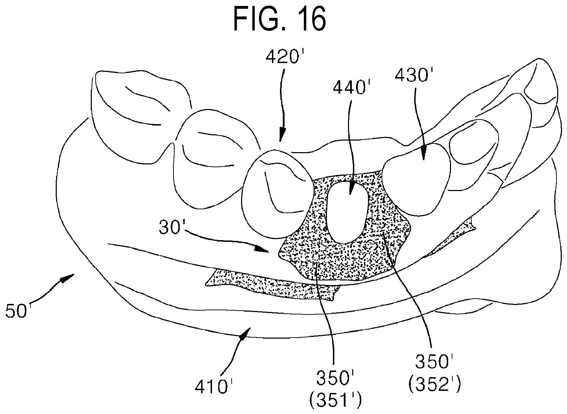

The above will be described with reference to scan data models. FIG. 13 illustrates a second scan data model 40', FIG. 14 illustrates a pre-scan data model 20', FIG. 15 illustrates an illustration of some of portions that are deleted when selectively merging the first scan data 30 and the second scan data 40, and FIG. 16 illustrates a final scan data model 50'.

Referring to FIG. 13, the second scan data model 40' includes a preparation tooth portion model 440', wherein a trimmed portion is displayed around the preparation tooth portion model 440'.

Referring to FIG. 14, the pre-scan data model 20' may be a model obtained by scanning an impression-taking cast, and data having a negative shape may be formed. A first scan data model (not shown) may be obtained by vertex normal conversion of the pre-scan data model 20', wherein at least a portion of the first scan data model includes the pre-trimming shape of an oral cavity, and may be combined with the second scan data model 40'.

Referring to FIG. 15, in the process of merging the first scan data model and the second scan data model 40', a portion with an intersection distance greater than or equal to the second threshold value may be shaded as an unnecessary portion O. Since the unnecessary portion O is a portion at which the rays generated from the first scan data do not meet the second scan data, or which is determined to be soft tissue or noise data, the first scan data of that portion may be deleted. Thus, the deletion of the first scan data in a region where the unnecessary portion O is formed has an advantage of preventing the precision of the final scan data from being reduced. In addition, the use of the present disclosure by the user has the advantage that the unnecessary portion O having an intersection distance greater than the second threshold value is automatically deleted, thereby improving user convenience, reducing work time, and saving system resources.

Referring to FIG. 16, the final scan data model 50' may be a model in which at least a portion of the first scan data model 30' is combined with the second scan data model 40'. For example, the final scan data model 50' may include normal tooth portion models 420' and 430' and the preparation tooth portion model 440' of the second scan data model 40', and a gingival complementary portion model 350' of the first scan data model 30' along an outer peripheral surface of the preparation tooth portion model 440'. Thus, the user can identify the intact shape of the gingival portion, if necessary, before trimming.

Meanwhile, the user can easily design the outer surface of a prosthesis along a gingival line by allowing only the gingival complementary model 350' to be displayed as needed. Further, by allowing only the second scan data model 40' to be displayed, the user can easily design a shoulder portion of the prosthesis along a margin line that appears in the second scan data model 40'. Alternatively, the user can identify the pre-trimming shape of the model by allowing all of the second scan data model 40' and selected portions of the first scan data model 30', including the gingival complementary portion model 350', to be displayed.

Hereinafter, a data processing method according to another embodiment of the present disclosure will be described in detail. In describing the data processing method according to the other embodiment of the present disclosure, the description redundant with that of the data processing method according to one embodiment of the present disclosure will be omitted or briefly made.

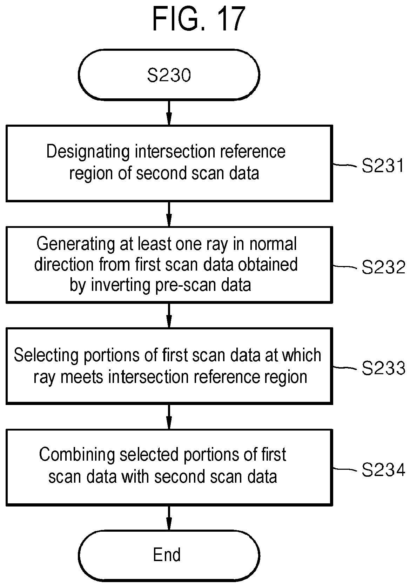

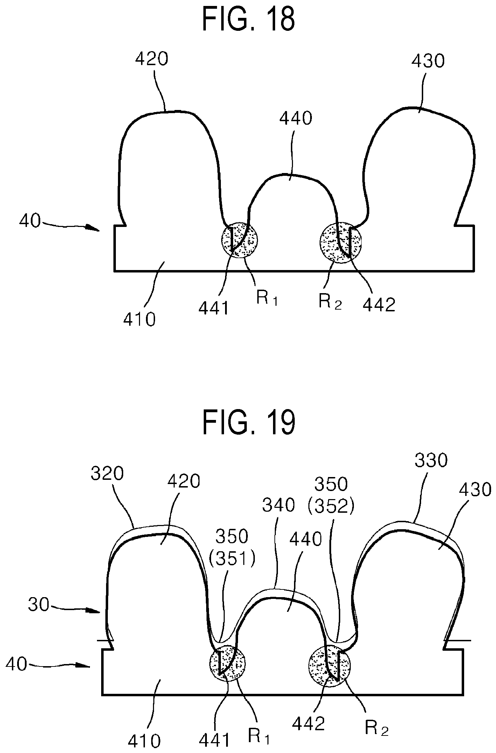

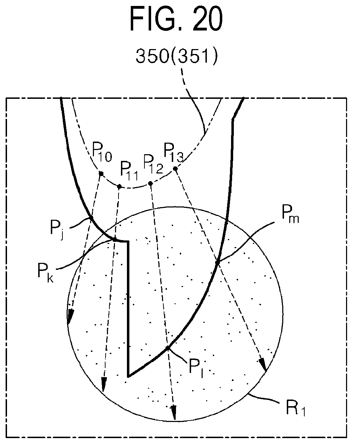

FIG. 17 is a detailed flowchart of operation S230 of a data processing method according to another embodiment of the present disclosure. FIG. 18 illustrates a process for designating an intersection reference region in the second scan data. FIG. 19 illustrates a process of aligning the first scan data with the second scan data. FIG. 20 illustrates a process of selecting a portion of the first scan data in the intersection reference region.

Referring to FIGS. 2, 12, and 17 to 20, the data processing method according to the other embodiment of the present disclosure may, like the above-described data processing method according to one embodiment of the present disclosure, obtain scan data from different casts (S 110) and align the obtained scan data (S120), but is distinguished from the one embodiment in the selectively merging operation S230.

In the data processing method according to the other embodiment of the present disclosure, the selectively merging operation S230 includes an operation S231 of designating an intersection reference region in the second scan data 40. For example, in the operation S231 of specifying the intersection reference region, intersection reference regions R1 and R2 of the second scan data 40 may be designated based on input from a user. Referring to FIG. 18, the user scans the trimmed second cast to obtain the second scan data 40. In order to obtain a gingival complementary portion of the second scan data 40 before trimming from the first scan data 30, the user may designate, as illustrated, regions that includes the trimmed portions 441 and 442. For example, the user may designate the first intersection designation region R1 including the first trimmed portion 441 and the second intersection designation region R2 including a second trimmed portion 442, wherein the first intersection designation region R1 and the second intersection designation region R2 are formed at both sides of the preparation tooth portion 440 in the second scan data 40.

Further, in the data processing method according to the other embodiment of the present disclosure, the selectively merging operation S230 may include an operation S232 of generating at least one ray in a normal direction from the first scan data 30 obtained by inverting the pre-scan data 20. The ray may be generated in a normal direction, which is a normal direction of the surface of the first scan data 30, and in a direction extending bidirectionally from the first scan data 30, and the process for generating the ray is the same as described above.

The selectively merging operation S230 may include an operation S233 of selecting portions of the first scan data 30 where rays meet the second scan data 40. More specifically, the operation S233 of selecting portions of the first scan data 30 may imply an operation of selecting portions where rays generated in the ray generation operation S232 meet the first scan data 30 when the rays meet the intersection reference regions R1 and R2. The selected portions of the first scan data 30 may correspond to the trimmed portions 441 and 442 of the second scan data 40, and may represent the pre-trimming shapes of the trimmed portions 441 and 442.

Referring to FIG. 19, the first scan data 30 and the second scan data 40 may be aligned, and it may be determined whether a ray generated from the first scan data 30 meets the second scan data 40. For example, referring to FIG. 20 together, four arbitrary points P10, P11, P12, and P13 may be generated in the gingival complementary portions 350 of the first scan data 30, and a ray may be generated at each point. The generated rays may meet points on the second scan data 40. In this case, a point Pj that meets the point P10 is outside the first intersection designation region R1, and a point Pk that meets the point P11, a point Pl that meets the point P12, and a point Pm that meets the point P13 are inside the first intersection designation region R1 designated by the user. Therefore, in the selection operation S232, the points of the first scan data 30, which meet the points Pk, Pl, and Pm existing inside the first intersection designation region R1 through the rays, may be selected, and the remaining portions of the first scan data 30 other than the selected data may be deleted. When the data processing method according to the other embodiment of the present disclosure is used, there is an advantage that only the gingival complementary portions 350 can be quickly and accurately selected without measuring the intersection distance and combined with the second scan data 40, and that the computation time of the system can be reduced to improve user convenience.

The selected portions of the first scan data 30 are combined to the second scan data (S233). The process of performing the combining operation S233 is the same as described above. The final scan data 50 obtained by combining the selected data may display the entire second scan data 40, the trimmed portions 441 and 442 of the second scan data 40, the gingival complementary portions 350 of the first scan data 30, and the entire final scan data 50 together or separately, depending on the user's needs.

The selected portions of the first scan data 30 may be portions between a first portion of the first scan data 30, at which a ray meets one end of each of the intersection reference regions R1 and R2, and a second portion of the first scan data 30, at which a ray meets the other end of each of the intersection reference regions R1 and R2. For example, a first point of the first scan data 30 which a ray meets may correspond to the left outermost portion of each of the intersection reference regions R1 and R2, and a second point of the first scan data 30 which a ray meets may correspond to the right outermost portion of each of the intersection reference regions R1 and R2. Thus, all of the portions (regions) between the first point of the first scan data 30 and the second point of the first scan data 30 may represent the gingival complementary portions 350. Accordingly, all of the portions of the first scan data 30 corresponding to the region between the first portion and the second portion may be selected and combined with the second scan data 40. This method is advantageous in that the final scan data 50 for accurately representing the gingival complementary portions 350 without any missing region between the first and second portions may be obtained and in that the user can provide optimal treatment to a patient.

The operation S120 of aligning the scan data 20, 30, and 40 and the selectively merging operations S130 and S230, described above, may be performed by a controller of a data processing device that will be described below. Further, at least some among the operation S110 of obtaining the scan data, the operation S120 of aligning the scan data, and the merging operations S130 and S230 may be visually displayed by a display unit of the data processing device which will be described below.

Hereinafter, a description will be made of a data processing device for performing the data processing method according to the one embodiment of the present disclosure and the data processing method according to the other embodiment of the present disclosure, described above.

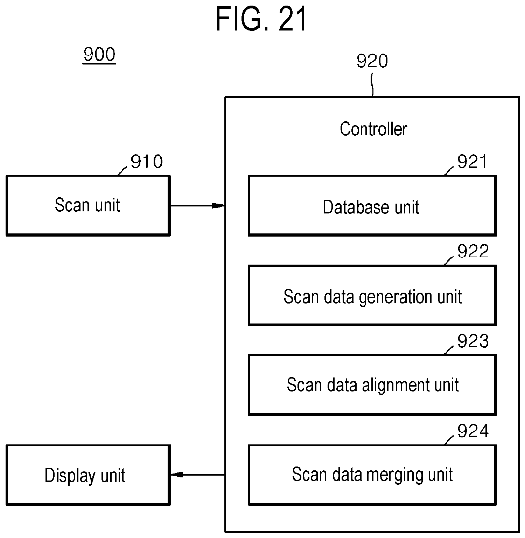

FIG. 21 schematically illustrates a configuration of a data processing device performing a data processing method according to the present disclosure.

Referring to FIG. 21, a data processing device 900 according to the present disclosure may include a scan unit 910, a controller 920, and a display unit 930. The scan unit 910 may obtain scan data by photographing different casts representing the oral cavity of a patient. The controller 920 may perform computational processing of the data, and may control the scan unit 910 and/or the display unit 930 as needed. The display unit 930 may display at least a portion of a process in which the data processing method according to the one or the other embodiment of the present disclosure is performed.

Hereinafter, each of elements of the data processing device 900 according to the present disclosure will be described.

The scan unit 910 may obtain scan data by photographing a cast. The scan unit 910 may be a three-dimensional scanner for obtaining two-dimensional and/or three-dimensional scan data by photographing a cast. For example, the scan unit 910 may be a handheld scanner. When the scan unit 910 is a handheld scanner, a user can hold the scan unit 910 in his/her hand and freely move the scan unit 910 with respect to a cast to photograph the cast from various distances and angles. The scan unit 910 may be a table scanner. The table scanner may include a tray on which a cast may be placed. When a user places a cast on the tray, the table scanner may rotate and/or tilt the tray, and may obtain scan data representing the cast by photographing the cast from multiple angles through a camera included in the table scanner.

The controller 920 may store the data obtained from the scan unit 910. For example, a database unit 921 of the controller 920 may store shots (scan shots) of a two-dimensional image or a three-dimensional image obtained by the scan unit 910 by photographing a cast. In addition, the database unit 921 may store sorting logic necessary to form scan data, scan data inversion logic for inverting pre-scan data, alignment logic for aligning different pieces of scan data, and logic necessary for selectively merging scan data.

A scan data generation unit 922 of the controller 920 may generate pre-scan data and second scan data by aligning and combining the shots (scan shots) of the two-dimensional image or the three-dimensional image obtained by the scan unit 910 by photographing the cast. In addition, the scan data generation unit 922 may obtain first scan data by inverting the pre-scan data by using the scan data inversion logic.

In addition, a scan data alignment unit 923 of the controller 920 may align the above-described scan data with each other. For example, the scan data alignment unit 923 may align the first scan data obtained by inverting the pre-scan data with the second scan data obtained by scanning a second cast. In this case, the scan data alignment unit 923 may align the first scan data with the second scan data such that the deviation between normal tooth portions and preparation tooth portions of the first scan data and the second scan data is minimized. In order to align the scan data, an ICP method may be used, but the present disclosure is not necessarily limited to using the ICP method to align scan data. The process of aligning the scan data by the scan data alignment unit 923 is the same as described above in relation to the data processing method according to the one embodiment of the present disclosure and the data processing method according to the other embodiment of the present disclosure, and a detailed description thereof will be omitted.

Further, a scan data merging unit 924 of the controller 920 may selectively merge the aligned scan data. For example, the scan data merging unit 924 may selectively merge a portion of the first scan data with the second scan data. More specifically, the scan data merging unit 924 may generate a ray in a normal direction from any point in the first scan data, and based on an intersection distance measured when the ray meets the second scan data, may delete at least a portion of the first scan data or combine the at least a portion of the first scan data with the second scan data. The first scan data and the second scan data may be combined by the scan data merging portion 924 to generate final scan data. The process of selectively merging the scan data by the scan data merging unit 924 is the same as described in operations S130 and S230 of the data processing method according to the one embodiment of the present disclosure and the data processing method according to the other embodiment of the present disclosure, and a detailed description thereof will be omitted.

The controller 920 described above may be configured to perform computational processing of data, and may be, for example, a computing device including a microprocessor. The controller 920 may be any one of devices capable of computing data, such as a PC, a server, and the like. Alternatively, the controller 920 may be a cloud capable of computational processing of data.

The data processing device 900 according to the present disclosure may further include a display unit 930. The display unit 930 may display at least some of the operations for performing the data processing method according to the one embodiment of the present disclosure and the data processing method according to the other embodiment of the present disclosure, described above. The display unit 930 may display a portion of the first scan data combined with the second scan data, the second scan data, and the final scan data. Further, the display unit 930 may display at least one among a process in which the scan unit 910 photographs a first cast to obtain the pre-scan data, a process in which the first scan data is obtained by inverting the pre-scan data, a process in which the second scan data is obtained by photographing the second cast, and a process in which the first scan data is aligned with the second scan data. The display unit 930 may be at least one of known visual display devices, including a monitor, a tablet, and a touch screen. A user can easily identify the data processing process displayed on the display unit 930, and can provide optimal treatment to a patient.

The above description is merely an exemplary description of the technical idea of the present disclosure, and a person skilled in the art, to which the present disclosure belongs, will appreciate that various modifications and variations are possible without departing from the essential features of the present disclosure.

Therefore, the embodiments described in the present disclosure are intended to explain and not to limit the technical idea of the present disclosure, and the scope of the technical idea of the present disclosure is not limited by these embodiments. The scope of the present disclosure shall be construed on the basis of the accompanying claims in such a manner that all of the technical ideas included within the scope equivalent to the claims belong to the present disclosure.

### INDUSTRIAL AVAILABILITY

The present disclosure provides a data processing method for selectively merging scan data, which facilitates identification of margin lines of a preparation tooth, or the like, with scan data that resembles an actual oral cavity, thereby obtaining final scan data that accurately represents the actual oral cavity of a patient.

## Claims

1. A data processing method comprising:
obtaining scan data from different casts;
aligning the obtained scan data; and
selectively merging a portion of one piece of the aligned scan data with another piece of the aligned scan data.

2. The data processing method of Claim 1, wherein the scan data comprises pre-scan data obtained by scanning a first cast, and second scan data obtained by scanning a second cast generated based on the first cast.

3. The data processing method of Claim 2, wherein the first cast is an impression-taking cast, and the second cast is an oral cavity cast.

4. The data processing method of Claim 3, wherein a part of a gingival portion of the second cast is formed by trimming.

5. The data processing method of Claim 2, wherein in the aligning, first scan data obtained by inverting the pre-scan data is aligned with the second scan data.

6. The data processing method of Claim 2, wherein the selectively merging comprises:
measuring an intersection distance between a predetermined region of the first scan data obtained by inverting the pre-scan data and a predetermined region of the second scan data;
selecting at least a portion of the first scan data based on the intersection distance; and
combining the data selected in the selecting,
wherein the selected data corresponds to a trimmed portion of the second scan data indicating a trimmed portion of the second cast.

7. The data processing method of Claim 6, wherein the intersection distance is a distance when a ray generated in a normal direction from one of the first scan data and the second scan data meets the other.

8. The data processing method of Claim 6, wherein in the selecting,
in case that the intersection distance is less than a first threshold value, the first scan data of a corresponding portion is deleted, and
in case that the intersection distance is equal to or greater than the first threshold value, the first scan data of a corresponding portion is selected.

9. The data processing method of Claim 6, wherein in the selecting,
in case that the intersection distance is less than the first threshold value or equal to or greater than a second threshold value, the first scan data of a corresponding portion is deleted, and
in case that the intersection distance is equal to or greater than the first threshold value and less than the second threshold value, the first scan data of a corresponding portion is selected.

10. The data processing method of one of Claims 8 and 9, wherein in the combining, the first scan data selected in the selecting and the second scan data are combined with each other to generate final scan data, and
wherein the final scan data is formed to display at least one of the selected first scan data and the second scan data.

11. The data processing method of Claim 2, wherein the selectively merging comprises:
designating an intersection reference region of the second scan data;
generating at least one ray in a normal direction from the first scan data obtained by inverting the pre-scan data;
selecting portions of the first scan data where the ray meets the intersection reference region; and
combining the second scan data with the portions of the first scan data selected in the selecting,
wherein the portions of the first scan data correspond to trimmed portions of the second scan data indicating trimmed portions of the second cast.

12. The data processing method of Claim 11, wherein in the combining, the portions of the first scan data selected in the selecting and the second scan data are combined with each other to generate final scan data, and
wherein the final scan data is formed to display at least one of the selected first scan data and the second scan data.

13. The data processing method of Claim 11, wherein the portions of the first scan data are portions between a first portion of the first scan data, at which the ray meets one end of the intersection reference region, and a second portion of the first scan data, at which the ray meets the other end of the intersection reference region.
